# EUROPÄISCHE PATENTSCHRIFT

(11) **EP 1 191 907 B1**
(45) Veröffentlichungstag und Bekanntmachung des Hinweises auf die Patenterteilung: **15.09.2004**
(21) Anmeldenummer: 00936878.8
(22) Anmeldetag: 13.06.2000
(51) Int. Cl.: A61F 2/34, A61F 2/46

(54) **MODULAR AUFGEBAUTE GELENKPFANNE FÜR EINE KUGELGELENK-PROTHESE**
MODULAR SOCKET FOR A BALL JOINT PROSTHESIS
CAVITE ARTICULAIRE MODULAIRE POUR PROTHESE DE ROTULE

(30) Priorität: 14.06.1999 DE 19926923
(43) Veröffentlichungstag der Anmeldung: 03.04.2002
(73) Patentinhaber: CeramTec AG Innovative Ceramic Engineering, 73207 Plochingen (DE)
(72) Erfinder: SILBERER, Paul, D-68753 Waghäusel (DE)
(74) Vertreter: Uppena, Franz, Dr.
(86) Internationale Anmeldenummer: PCT/EP2000/005427
(87) Internationale Veröffentlichungsnummer: WO 2000/076427

(56) Entgegenhaltungen:
- EP-A- 0 091 315
- EP-A- 0 142 759
- EP-A- 0 551 794
- EP-A- 0 694 294
- EP-A- 0 811 360
- EP-A- 0 853 928
- DE-A- 4 335 931
- DE-A- 4 402 675
- DE-A- 19 611 249
- DE-C- 19 708 604
- US-A- 5 092 897
- US-A- 5 658 338
- US-A- 5 725 591
- US-A- 5 766 260

## Beschreibung

Die Erfindung betrifft eine modular aufgebaute Gelenkpfanne für eine Kugelgelenk-Prothese zum Einsetzen in Knochengewebe.

Eine Prothese gemäß dem Oberbegriff von Anspruch 1 ist aus der EP-A-06 94 294 bekannt.

Modular aufgebaute Gelenkpfannen für Kugelgelenk-Prothesen bestehen in der Regel aus einer äußeren, in den Knochen einzusetzenden Metallschale als Pfannengehäuse und einer in das Pfannengehäuse eingesetzten, inneren Gleitschale. Diese Gleitschale kann beispielsweise aus Keramik bestehen und mit Hilfe einer konischen Klemmung in der Metallschale, dem Pfannengehäuse, fixiert sein. Eine so aufgebaute Pfanne ist beispielsweise aus der DE 43 35 931 A1 bekannt. Aus der DE 44 02 675 A1 ist eine Gelenkpfanne bekannt, bei der die innere Gleitschale ohne konische Klemmung in das Pfannengehäuse eingesetzt ist. Damit die Gleitschale gegen Verdrehen und Herausfallen geschützt ist, sich jedoch zerstörungsfrei aus ihrem Sitz herausdrücken und auswechseln läßt, wird sie über einen Haltering in der Metallschale fixiert, der mit Schrauben auf der Stirnseite des Pfannengehäuses festgeschraubt ist.

Bei den bekannten Ausführungsformen wird der äußere Rand der in den Knochen einzusetzenden Metallschale, der Rand des Pfannengehäuses, versteift. Das Pfannengehäuse verliert seine Elastizität. Als Folge kann dadurch eine verstärke Migration der Gelenkpfanne im Knochen auftreten. Das führt zur allmählichen Lockerung des Implantats im Knochen.

Ein weiteres Problem ist die Anpassung der Gleitschale an die Abmessung des Pfannengehäuses. Die Abmessungen der Gelenkpfannen, im wesentlichen der Durchmesser des Kugelkopfs und daraus folgernd des Innendurchmessers der Gleitschale, werden im wesentlichen auf das Alter des Patienten und seine Körperstatur abgestimmt. Insbesondere bei der konischen Klemmpassung muß die äußere Kontur der Gleitschale sehr genau auf die innere Kontur des Pfannengehäuses abgestimmt sein. Bei eventuellen Nachoperationen ist es deshalb von Vorteil, wenn von der Endoprothese im Oberschenkel zumindest der Schaft und in dem Beckenknochen zumindest das Pfannengehäuse verbleiben kann und nur der Kugelkopf der Kugelgelenk-Prothese und die Gleitschale ausgewechselt werden müssen.

Die Aufgabe der vorliegenden Erfindung besteht darin, den Einsatz einer Gleitschale aus Keramik in ein Pfannengehäuse aus Metall zu verbessern und die Entnahme der Gleitschale aus dem Pfannengehäuse bei einer eventuellen erneuten Operation zu erleichtern.

Die Lösung der Aufgabe erfolgt mit Hilfe der kennzeichnenden Merkmale des ersten Anspruchs. Vorteilhafte Ausgestaltungen der Erfindung werden in den Unteransprüchen beansprucht, wozu ein Werkzeug gehört, das in vorteilhafter Weise geeignet ist, eine Gleitschale zu handhaben.

Die erfindungsgemäße, modular aufgebaute Gelenkpfanne für eine Kugelgelenk-Prothese unterscheidet sich von den herkömmlichen Prothesen dadurch, daß die Gleitschale aus Keramik nicht direkt in das Pfannengehäuse eingesetzt ist. Die Gleitschale aus Keramik ist fest in einen Ring aus einem biokompatiblen Metall eingesetzt und erst dieser Ring wird in das Pfannengehäuse eingesetzt. Zur Befestigung des Ringes mit der Gleitschale sind mindestens drei sich radial nach außen erstreckende Befestigungselemente am Ring vorgesehen. Diese Befestigungselemente greifen jeweils in eine Ausnehmung in dem Pfannengehäuse ein. Der Ring liegt aber nicht direkt am Pfannengehäuse an, sondern ist von diesem durch einen Spalt beabstandet. Durch die erfindungsgemäße Befestigung der Gleitschale wird verhindert, daß durch die eingesetzte Gleitschale das Pfannengehäuse versteift wird, wie es bei einer herkömmlichen konischen Flächenklemmung zwischen Gleitschale und Pfannengehäuse der Fall ist. Eine durch Versteifung mögliche Migration des Pfannengehäuses wird dadurch vermieden. Das Pfannengehäuse behält seine Elastizität. Weiterhin wird vermieden, daß bei unsachgemäßem oder unvorsichtigem Einsetzen der Gleitschale in das Pfannengehäuse Randabplatzungen an der Gleitschale auftreten, die die Prothese unbrauchbar machen. Dem Chirurgen wird die Arbeit erleichtert, da das Einsetzen der Gleitschalen in die Pfannengehäuse unter Operationsbedingungen erleichtert wird.

Die Verbindung zwischen der Gleitschale und dem Ring kann entweder durch konische Klemmung oder durch Aufschrumpfen des Rings erfolgen. Beim Aufschrumpfen wird der Ring erwärmt und über die Gleitschale gezogen. Beim Abkühlen entsteht dadurch eine Schrumpfverbindung.

Damit ein einwandfreier Sitz der Gleitschale in dem Pfannengehäuse gewährleistet ist und das Pfannengehäuse gleichmäßig belastet wird, ist es von Vorteil, wenn der Spalt zwischen Gleitschale und Pfannengehäuse überall die gleichen Abmessungen aufweist. Um einen konstanten Abstand zwischen Gleitschale und Pfannengehäuse herzustellen und zu gewährleisten, weisen die Befestigungselemente Abstandshalter auf. Die Abstandshalter können außerdem so ausgebildet sein, daß sie durch eine Abstützung auf der Wand des Pfannengehäuses im Bereich der Befestigungselemente eine gleichmäßige Einleitung der Kraft in das Pfannengehäuse gewährleisten. Je nach Größe des Pfannengehäuses und der Gleitschale liegen die Abstände zwischen Gleitschale und dem Pfannengehäuse zwischen etwa 1 mm und 1,8mm.

Um ein sichere Verankerung des Rings mit der Gleitschale in dem Pfannengehäuse zu gewährleisten, verbreitern sich die Befestigungselemente in radialer Richtung keilförmig und die Ausnehmungen in dem Pfannengehäuse, in die die Befestigungselemente eingreifen, sind der Kontur der Befestigungselemente angepaßt. Bei einer Druckbelastung auf die Gleitschale wird diese versuchen, das Pfannengehäuse auseinanderzudrücken. Durch die keilförmige Verbreiterung in radialer Richtung wird dieses Auseinanderdrücken verhindert und die Befestigungselemente der Gleitschale halten durch ihre Klemmwirkung das Pfannengehäuse in seiner Form, so das ein Herausfallen der Gleitschale aus dem Pfannengehäuse aufgrund einer Aufweitung vermieden wird.

Die Krafteinleitung über die Befestigungselemente in das Pfannengehäuse wird weiterhin dadurch verbessert, wenn die Befestigungselemente sich in Richtung des Einsetzens in das Pfannengehäuse keilförmig verjüngen und die Ausnehmungen in dem Pfannengehäuse der Kontur der Befestigungselemente angepaßt sind. Durch die Formgebung der Ausnehmungen wird eine einseitige Krafteinleitung über die Befestigungselemente in Richtung der Meridiane in das Pfannengehäuse vermieden, das in der Regel im wesentlichen halbkugelförmig geformt ist oder die Form eines Kugelabschnitts aufweist. Durch die keilförmige Verjüngungen der Befestigungselemente treten nicht nur Kraftkomponenten in Richtung der Meridiane im Bereich der kugelförmigen Fläche auf, sondern auch senkrecht dazu, in Richtung der Breitenkreise des Pfannengehäuses, so daß eine Vergleichmäßigung der Kraftanleitung im Bereich der Ausnehmungen im Pfannengehäuse gewährleistet ist.

Aufgrund unterschiedlicher Bauarten des Pfannengehäuses bestehen unterschiedliche Innendurchmesser zur Aufnahme von Gleitschalen, die ein und denselben Innendurchmesser der Gleitfläche aufweisen, also auf ein und dieselbe Kugelkopfgröße abgestimmt sind. Deshalb besteht ein weiterer Vorteil der Einbettung der Gleitschalen in Ringe darin, daß der Außendurchmesser der Gleitschalen nicht mehr auf die unterschiedlichen Bauarten der Pfannengehäuse abgestimmt werden muß. Es genügt, eine Gleitschale mit einem vorgegebenen Außendurchmesser herzustellen und durch Ringe mit unterschiedlicher Dicke die Gleitschalen jeweils an die unterschiedlichen Bauarten der Pfannengehäuse anzupassen. Der Erfindung liegt damit die Idee zugrunde, daß es einfacher ist, eine Gleitschale aus keramischen Werkstoff nur in einer Größe herzustellen, weil dadurch die Vorhaltung mehrerer Formen und die entsprechende Nachbearbeitung der Gleitschalen mit unterschiedlichem Werkzeug und unterschiedlichen Maschineneinstellungen überflüssig wird. Für eine Gleitschale, die für einen Kugelkopf mit einem Durchmesser von 28 mm vorgesehen ist, verringert sich durch die erfindungsgemäße Einbettung der Gleitschalen in Ringe die Anzahl der vorzuhaltenden Baugrößen um sechs, für einen Kugelkopfdurchmesser von 32 mm um drei.

Als weitere vorteilhafte Ausgestaltung der Erfindung ist ein Werkzeug zur Handhabung der mit Ring versehenen Gleitschalen vorgesehen. Mit ihm ist das Einsetzen einer Gleitschale in das und das Herausnehmen aus dem Pfannengehäuse möglich. Das Werkzeug besitzt zwei axial gegeneinander verschiebbare Komponenten, wobei sich die erste Komponente, die Abstützeinrichtung, auf den Rand des Pfannengehäuses abstützt und die zweite Komponente, die Druck- und Zugeinrichtung, in Abhängigkeit der Handhabung der Gleitschale an der Gleitschale positionierbar ist. Die Druck- und Zugeinrichtung ist so konstruiert, daß sie zum Einsetzen der Gleitschale in das Pfannengehäuse auf die Befestigungselemente aufsetzbar ist, um dann durch eine entsprechende Handhabung eine Druckkraft auf die Befestigungselemente auszuüben, die in Richtung des Pfannengehäuses weist und dadurch die Gleitschale in das Pfannengehäuse einsetzt, wobei die Befestigungselemente in die entsprechenden Ausnehmungen der Gleitschale eingedrückt werden.

Um das Lösen an der Gleitschale aus dem Pfannengehäuse zu ermöglichen, ist die Druck- und Zugeinrichtung weiterhin so ausgestaltet, daß sie die Befestigungselemente hintergreifen kann und durch Gegeneinanderbewegen der beiden Komponenten des Werkzeugs eine in Richtung aus dem Pfannengehäuse weisende Zugkraft auf die Gleitschale ausübbar ist. Während sich die Abstützeinrichtung auf dem Rand des Pfannengehäuses abstützt, zieht die Druck- und Zugeinrichtung die Befestigungselemente aus den Ausnehmungen in dem Pfannengehäuse. Das Werkzeug ist von seiner Funktionsweise her mit einer Abziehvorrichtung von Lagern von Wellen vergleichbar, aber mit dem Unterschied, daß hier nicht die Abstützung auf eine zentral angeordnete Welle erfolgt und das abzuziehende Objekt um den Abstützpunkt herum angeordnet ist, sondern daß sich das zu entfernende Objekt innerhalb des Gegenstandes befindet, aus dem es herausgezogen werden soll.

Anhand eines Ausführungsbeispiels wird die Erfindung näher erläutert.

Es zeigen:
- Figur 1: eine Aufsicht auf eine erfindungsgemäße, modular aufgebaute Gelenkpfanne,
- Figur 2: einen Meridian-Schnitt durch die Gelenkpfanne, entsprechend dem Schnittverlauf in Figur 1,
- Figur 3: als Einzelheit den Schnitt durch ein Befestigungselement in der Wand des Pfannengehäuses und
- Figur 4: ein Werkzeug zur Handhabung von Gleitschalen.

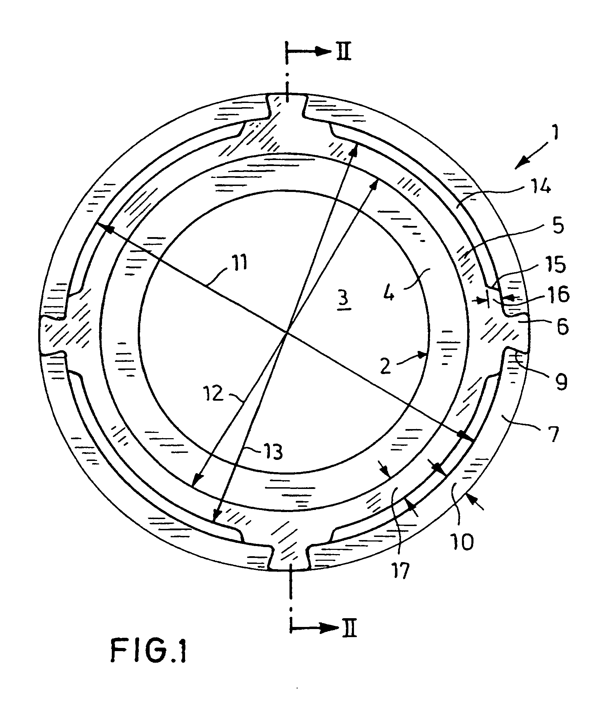

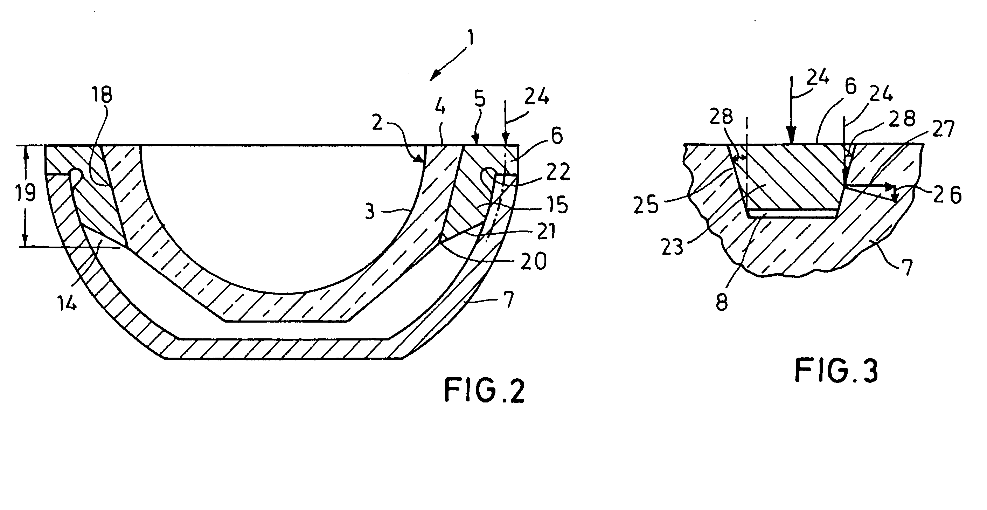

In Figur 1 ist im vergrößertem Maßstab die Aufsicht auf eine erfindungsgemäße, modular aufgebaute Gelenkpfanne 1 dargestellt. Von der Gleitschale 2 ist die Gleitfläche 3 und die Stirnfläche 4 zu sehen. Die Gleitschale 2 wird von einem Ring 5 aus einem biokompatiblen Metall umgeben. Der Ring 5 weist im vorliegenden Ausführungsbeispiel vier in gleichmäßigen Abständen auf dem Umfang verteilte Befestigungselemente 6 auf. Die Befestigungselemente 6 sind in dem Pfannengehäuse 7 verankert. Sie greifen dort in entsprechend geformte Ausnehmungen 8 (Figur 3) ein. Die Aufsicht auf die Befestigungselemente 6 zeigt deren sich keilförmig nach außen verbreiternde Form 9. Das Pfannengehäuse 7 weist eine seiner Bauart entsprechende Wandstärke 10 auf, die zwischen 2 mm und 5 mm liegen kann. Mit 11 ist der Innendurchmesser des Pfannengehäuses 7 zur Aufnahme der Gleitschale 2 bezeichnet. Wie ersichtlich, ist dieser Innendurchmesser 11 wesentlich größer als der Außendurchmesser 12 der Gleitschale 2. Er ist weiterhin größer als der Außendurchmesser 13 des Rings 5. Dadurch entsteht zwischen dem Ring 5 und dem Pfannengehäuse 7 ein Spalt 14. Um eine gleichmäßige Breite des Spalts 14 zu gewährleisten, weisen die Befestigungselemente 6 sogenannte Abstandshalter 15 auf. Die Abstandshalter 15, aus demselben Material wie der Ring 5 und die Befestigungselemente 6, haben eine auf die Bauart des Pfannengehäuses 7 abgestimmte Dicke 16, die der gewünschten Spaltbreite entspricht. Die Abstandshalter 15 dienen nicht nur zu einer gleichmäßigen Ausbildung des Spalts 14, sondern sorgen auch für eine gleichmäßige Abstützung des Rings 5 und damit der Gleitschale 2 in dem Pfannengehäuse 7.

Gemäß der Erfindung ist der Außendurchmesser 12 einer Gleitschale 2 mit einer Gleitfläche 3 vorgegebenen Durchmessers für alle Bauarten eines Pfannengehäuses 7 konstant. Durch entsprechende Wandstärken 17 der Ringe 5 wird der Unterschied zwischen dem Innendurchmesser 11 eines Pfannengehäuses 7 und dem Außendurchmesser 12 einer Gleitschale 2 ausgeglichen, so daß der jeweilige Spalt 14, der in seiner Breite den Abmessungen 16 der Abstandshalter 15 entspricht, bei allen Bauarten der Pfannengehäuse im wesentlichen gleich groß ist. Der Spalt 14 hat eine Breite 16, die auf den Durchmesser der Gleitfläche und die Bauart des Pfannengehäuses 7 abgestimmt, zwischen etwa 1 mm und etwa 1,8 mm liegt. Die Wandstärke 17 der Ringe 5 reicht deshalb von etwa 2 mm bis etwa 5 mm.

Figur 2 zeigt einen Schnitt durch die erfindungsgemäße, modular aufgebaute Gelenkpfanne 1 entsprechend dem in der Figur 1 eingezeichneten Schnittverlauf II-II. Der Schnitt verläuft durch zwei gegenüberliegende Befestigungselemente 6. Wie aus der Figur 2 ersichtlich ist, umschließt der Ring 5 die Gleitschale 2 in ihrem äußeren Bereich 18, wo bei herkömmlichem Einsatz einer Gleitschale in ein Pfannengehäuse die Klemmung mit dem Pfannengehäuse erfolgt. Die Verbindung zwischen dem Ring 5 und der Gleitschale 2 kann dem Stand der Technik vergleichbar mittels konischer Klemmung oder durch Aufschrumpfen erfolgen. Die Höhe 19 des Rings 5 liegt, in Anlehnung an die herkömmliche konische Klemmung in einem Pfannengehäuse, etwa bei 10 mm. Um bei der Klemmung der Gleitschale 2 im Ring 5 Spannungen an der Übergangsstelle 20 zwischen Ring 5 und Gleitschale 2 zu vermeiden, verjüngt sich der Ring 5 keilförmig 21 zur Gleitschale 2 hin.

Das vorliegende Ausführungsbeispiel zeigt, wie der Ring 5 mit seinen Abstandshaltern 15 an dem Pfannengehäuse 7 anliegt. Weiterhin ist unter dem Befestigungselement 6 eine Kerbe 22 zu sehen, in die, hier nicht dargestellt, Greifer eines Werkzeugs eingreifen können, um den Ring 5 mit der Gleitschale 2 aus dem Pfannengehäuse 7 herauszuheben.

Figur 3 zeigt den Eingriff eines Befestigungselements 6 in die Wand des Pfannengehäuses 7. Es ist eine Schnitt-Darstellung in einem nochmals vergrößerten Maßstab. Das Befestigungselement 6 hat einen trapezförmigen Querschnitt 23. Die Ausnehmung 8, in die das Befestigungselement 6 eingreift, hat eine dem Querschnitt 23 des Befestigungselements 6 angepaßte Form. Der trapezförmige Querschnitt 23 verjüngt sich in Richtung 24 der auf die Gleitschale 2 einwirkenden Kräfte und der Meridiane des Pfannengehäuses 7 (Fig. 2), das im vorliegenden Ausführungsbeispiel angenähert die Form einer Kugelzone aufweist.

Aufgrund der schräg zur Wirkrichtung 24 der Kräfte verlaufenden Seitenlinien 25 der trapezförmigen Befestigungselemente 6 erfolgt die Krafteinleitung in das Pfannengehäuse 7 mit einer Komponente 26 in meridianer Richtung 24 und mit einer Komponente 27 in einer senkrecht dazu verlaufenden Richtung, entsprechend der Breitenkreise. Die Seitenwände 25 sind im vorliegenden Ausführungsbeispiel gegenüber den Meridianen und der Hauptwirkrichtung der Kräfte 24 in einem Winkel 28 von etwa 15 ° geneigt.

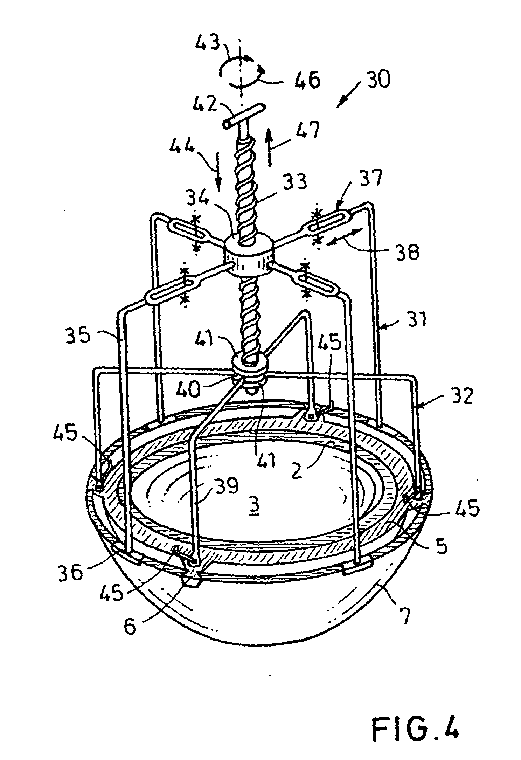

In Figur 4 ist ein Werkzeug 30 dargestellt, mit dem eine in einem Ring eingebettete Gleitschale 2 in ein Pfannengehäuse 7 eingesetzt oder wieder aus ihm entfernt werden kann. Die Darstellung des Werkzeugs 13 ist rein schematisch und soll nur seine Wirkungsweise erläutern.

Das Werkzeug 30 weist zwei axial gegeneinander verschiebbare Komponenten auf, eine Abstützeinrichtung 31 und eine Druck- und Zugeinrichtung 32, die über eine Gewindespindel 33 miteinander verbunden sind, wobei die Abstützeinrichtung 31 an einer Mutter 34 befestigt ist, die auf der Gewindespindel 33 bewegbar angeordnet ist, und die Druck- und Zugeinrichtung 32 an der Gewindespindel selbst frei drehbar befestigt ist.

Die Abstützeinrichtung 31 besteht im vorliegenden Ausführungsbeispiel aus vier parallel zueinander und parallel zur Gewindespindel 33 angeordneten Stützen 35. Die Stützen 35 haben, auf den Umfang der Mutter 34 gesehen, gleich große Winkelabstände. Sie stützen sich mit Füßen 36 auf der Stirnfläche des Pfannengehäuses 7 ab. Eine hier nicht in Einzelheiten dargestellte Verstelleinrichtung 37 ermöglicht die Anpassung der Stützen an unterschiedliche Durchmesser der Pfannengehäuse 7. Das Verstellen kann beispielsweise durch Lösen der hier angedeuteten Schrauben und durch Verschieben der Stützen entlang von Langlöchern entsprechend dem Doppelpfeil 38 vorgenommen werden.

Die vier Stützen 39 der Druck- und Zugeinrichtung 32 sind in ihrer Anzahl und Anordnung auf die Befestigungselemente 6 des Rings 5 abgestimmt. Sie sind starr über einen Ring 40 miteinander verbunden und jeweils zwischen den Stützen 35 der Abstützeinrichtung 31 angeordnet. Der Ring wird frei drehbar am Ende der Gewindespindel 33 zwischen zwei Haltescheiben 41 gehalten.

Im vorliegenden Ausführungsbeispiel ist der Vorgang des Einsetzens einer Gleitschale 2 in ein Pfannengehäuse 7 dargestellt. Die Stützen 39 der Druck- und Zugeinrichtung 32 sind jeweils auf die Befestigungselemente 6 aufgesetzt. Die Stützen 35 der Abstützeinrichtung 31 stützen sich auf dem Rand des Pfannengehäuses 7 ab. Durch Drehen an dem Handknebel 42 am oberen Ende der Gewindespindel 33 im Uhrzeigersinn 43 bewegen sich die Stützen 39 der Druck- und Zugeinrichtung 32 in Richtung 44 und drücken damit die Befestigungselemente 6 in die Ausnehmungen 8 des Pfannengehäuses 7. Damit dieses Eindrücken erfolgen kann, muß auf die Abstützeinrichtung 31 ebenfalls in Pfeilrichtung 44 ein Druck ausgeübt werden. Die an den Enden der Stützen 39 gelenkig angeordneten Haken 45 zum Ausheben der Gleitschalen sind hochgeschwenkt, damit sie das Eindrücken der Gleitschale 2 nicht behindern.

Soll eine Gleitschale 2 aus einem Pfannengehäuse 7 entfernt werden, so werden zunächst die an den Enden der Stützen 39 gelenkig angeordneten Haken 45 unter die Befestigungselemente 6 geschoben, so daß sie in die Kerben 22 eingreifen. Wird dann die Gewindespindel 33 im Gegenuhrzeigersinn 46 gedreht, während sich die Abstützeinrichtung 31 auf dem Rand des Pfannengehäuses 7 abstützt, wird die Druckund Zugeinrichtung 32 in Pfeilrichtung 47 bewegt und hebt damit die Befestigungselemente 6 aus dem Pfannengehäuse 7 und entfernt damit die Gleitschale 2 aus dem Pfannengehäuse 7.

## Patentansprüche

1. Modular aufgebaute Gelenkpfanne für eine Kugelgelenk-Prothese zum Einsetzen in Knochengewebe, bestehend aus einer äußeren, in den Knochen einzusetzenden Metallschale als Pfannengehäuse (7) und eine in das Pfannengehäuse eingesetzte, innere Gleitschale (2) aus Keramik, wobei die Gleitschale (2) aus Keramik fest in einen Ring (5) aus einem biokompatiblen Metall eingesetzt ist, **dadurch gekennzeichnet, daß** der Ring (5) mit mindestens drei sich radial nach außen erstreckenden Befestigungselementen (6) besetzt ist, daß die Befestigungselemente (6) jeweils in eine Ausnehmung (8) in dem Pfannengehäuse (7) eingreifen und daß der Ring (5) von dem Pfannengehäuse (7) durch einen Spalt (14) beabstandet ist.

2. Modular aufgebaute Gelenkpfanne nach Anspruch 1, **dadurch gekennzeichnet, daß** die Gleitschale (2) durch eine konische Klemmung in dem Ring (5) befestigt ist.

3. Modular aufgebaute Gelenkpfanne nach Anspruch 1, **dadurch gekennzeichnet, daß** der Ring (5) auf die Gleitschale (2) aufgeschrumpft ist.

4. Modular aufgebaute Gelenkpfanne nach einem der Ansprüche 1 bis 3, **dadurch gekennzeichnet, daß** zur Bildung eines Spalts (14) mit konstantem Abstand zwischen Gleitschale (2) und dem Pfannengehäuse (7) die Befestigungselemente (6) Abstandshalter (15) aufweisen.

5. Modular aufgebaute Gelenkpfanne nach einem der Ansprüche 1 bis 4, **dadurch gekennzeichnet, daß** sich die Befestigungselemente (6) am Ring (5) in radialer Richtung keilförmig verbreitern (9) und daß die Ausnehmungen (8) in dem Pfannengehäuse (7) der Kontur der Befestigungselemente (6) angepaßt sind.

6. Modular aufgebaute Gelenkpfanne nach einem der Ansprüche 1 bis 5, **dadurch gekennzeichnet, daß** die Befestigungselemente (6) sich in Richtung (24) des Einsetzens in das Pfannengehäuse (7) keilförmig verjüngen (23) und daß die Ausnehmungen (8) in dem Pfannengehäuse (7) der Kontur der Befestigungselemente (6) angepaßt sind.

7. Modular aufgebaute Gelenkpfanne nach einem der Ansprüche 1 bis 6, **dadurch gekennzeichnet, daß** der Außendurchmesser (12) der Gleitschale (2) konstant ist und daß bei unterschiedlichen Innendurchmessern (11) des Pfannengehäuses (7) zur Aufnahme der Gleitschale (2) der Außendurchmesser (13) des Rings (5) dem Innendurchmesser (11) des Pfannengehäuses (7) so angepaßt ist, daß die Abmessungen des Spalts (14) bei den möglichen Kombinationen von Gleitschale (2) und Pfannengehäuse (7) stets in etwa gleich groß ist.

8. Modular aufgebaute Gelenkpfanne nach einem der Ansprüche 1 bis 7, **dadurch gekennzeichnet, daß** ein Werkzeug (30) zur Handhabung der in einen Ring (5) eingesetzten Gleitschale (2) vorgesehen ist zum Einsetzen in das und Herausnehmen aus dem Pfannengehäuse (7)

9. Modular aufgebaute Gelenkpfanne nach Anspruch 8, **dadurch gekennzeichnet, daß** das Werkzeug (30) zwei axial gegeneinander verschiebbare Komponenten (31, 32) aufweist, daß sich die erste Komponente als Abstützeinrichtung (31) auf dem Rand des Pfannengehäuses (7) abstützt und daß die zweite Komponente als Druck- und Zugeinrichtung (32), in Abhängigkeit der vorgesehenen Handhabung der Gleitschale (2), an den Befestigungselementen (6) der Gleitschale (2) positionierbar ist.

10. Modular aufgebaute Gelenkpfanne nach Anspruch 9, **dadurch gekennzeichnet, daß** zum Einsetzen der Gleitschale (2) in das Pfannengehäuse (7) die Druckund Zugeinrichtung (32) auf die Befestigungselemente (6) und die Abstützeinrichtung (31) auf dem Rand des Pfannengehäuses (7) aufsetzbar sind und daß durch ein Verschieben der beiden Komponenten (31, 32) des Werkzeugs (30) voneinander weg auf die Gleitschale (2) eine Druckkraft in Richtung (44) des Pfannengehäuses (7) ausübbar ist.

11. Modular aufgebaute Gelenkpfanne nach Anspruch 9, **dadurch gekennzeichnet, daß** zum Herauslösen einer Gleitschale (2) aus dem Pfannengehäuse (7) die Abstützeinrichtung (31) auf dem Rand des Pfannengehäuses (7) aufsetzbar ist und die Befestigungselemente (6) mittels der Druck- und Zugeinrichtung (32) hintergreifbar sind und daß durch Verschieben der beiden Komponenten (31, 32) des Werkzeugs (30) aufeinander zu die Gleitschale (2) aus dem Pfannengehäuse (7) heraushebbar ist.

## Claims

1. Modularly constructed joint socket for a ball-joint prosthesis for insertion into bone tissue, consisting of an outer metal cup as a socket shell (7) which is to be inserted into the bone and an inner sliding cup (2) made from ceramic material that is inserted into the socket shell, with the sliding cup (2) that is made from ceramic material being fixedly inserted into a ring (5) made from a biocompatible metal, **characterised in that** the ring (5) has at least three securing elements (6) extending radially outwards, **in that** the securing elements (6) engage into respective recesses (8) in the socket shell (7), and **in that** the ring (5) is spaced apart from the socket shell (7) by a gap (14).

2. Modularly constructed joint socket according to claim 1, **characterised in that** the sliding cup (2) is secured in the ring (5) by conical clamping.

3. Modularly constructed joint socket according to claim 1, **characterised in that** the ring (5) is shrunk onto the sliding cup (2).

4. Modularly constructed joint socket according to one of claims 1 to 3, **characterised in that** in order to form a gap (14) with constant spacing between the sliding cup (2) and the socket shell (7) the securing elements (6) have spacers (15).

5. Modularly constructed joint socket according to one of claims 1 to 4, **characterised in that** the securing elements (6) on the ring (5) widen out (9) in a radial direction in a wedge-shaped manner, and **in that** the recesses (8) in the socket shell (7) are matched to the contours of the securing elements (6).

6. Modularly constructed joint socket according to one of claims 1 to 5, **characterised in that** the securing elements (6) taper (23) in a wedge-shaped manner in the direction (24) of insertion into the socket shell (7), and **in that** the recesses (8) in the socket shell (7) are matched to the contours of the securing elements (6).

7. Modularly constructed joint socket according to one of claims 1 to 6, **characterised in that** the external diameter (12) of the sliding cup (2) is constant, and **in that**, in the case of varying internal diameters (11) of the socket shell (7) for receiving the sliding cup (2), the external diameter (13) of the ring (5) is matched to the internal diameter (11) of the socket shell (7) in such a way that the dimensions of the gap (14) in the possible combinations of sliding cup (2) and socket shell (7) is (*sic*) always substantially of the same magnitude.

8. Modularly constructed joint socket according to one of claims 1 to 7, **characterised in that** a tool (30) for the manipulation of the sliding cup (2) that is inserted into a ring (5) is provided for the purposes of insertion into and removal from the socket shell (7).

9. Modularly constructed joint socket according to claim 8, **characterised in that** the tool (30) has two components (31, 32) which are axially displaceable in relation to each other, **in that** the first component as a supporting device (31) is supported on the edge of the socket shell (7), and **in that** the second component as a push-pull device (32) can be positioned on the securing elements (6) of the sliding cup (2) according to the intended manipulation of the sliding cup (2).

10. Modularly constructed joint socket according to claim 9, **characterised in that** for the purpose of inserting the sliding cup (2) into the socket shell (7), the push-pull device (32) can be placed on the securing elements (6), and the supporting device (31) can be placed on the edge of the socket shell (7), and **in that**, by displacing the two components (31, 32) of the tool (30) away from each other, a pressure force can be exerted on the sliding cup (2) in the direction (44) of the socket shell (7).

11. Modularly constructed joint socket according to claim 9, **characterised in that** for the purpose of extracting a sliding cup (2) out of the socket shell (7), the supporting device (31) can be placed on the edge of the socket shell (7), and the push-pull device (32) can engage behind the securing elements (6), and **in that**, by displacing the two components (31, 32) of the tool (30) towards each other, the sliding cup (2) can be lifted out from the socket shell (7).

## Revendications

1. Cotyle modulaire pour une prothèse de rotule destinée à être implantée dans le tissu osseux, comprenant une coquille extérieure en métal à implanter dans l'os en tant que corps de cotyle (7) et une cupule (2) intérieure en céramique à insérer dans le corps de cotyle, la cupule (2) en céramique étant montée serrée dans une bague (5) en un métal biocompatible, **caractérisée par le fait que** la bague (5) est pourvue d'au moins trois éléments de fixation (6), qui s'étendent radialement vers l'extérieur, que les éléments de fixation (6) s'engagent chacun dans un évidement (8) aménagé dans le corps de cotyle (7) et que la bague (5) est séparée du corps de cotyle (7) par un interstice (14).

2. Cotyle modulaire de rotule selon la revendication 1, **caractérisée par le fait que** la cupule (2) est fixée dans la bague (5) par un emmanchement conique.

3. Cotyle modulaire de rotule selon la revendication 1, **caractérisée par le fait que** la bague (5) est frettée sur la cupule (2).

4. Cotyle modulaire de rotule selon une des revendications 1 à 3, **caractérisée par le fait que** pour former un interstice (14) avec une distance constante entre la cupule (2) et le corps de cotyle (7), les éléments de fixation (6) comportent des moyens d'espacement (15).

5. Cotyle modulaire de rotule selon une des revendications 1 à 4, **caractérisée par le fait que** les éléments de fixation (6) sur la bague (5) s'élargissent en forme de coin, dans la direction radiale (9), et que les évidements (8) dans le corps de cotyle (7) sont adaptés au contour des éléments de fixation (6).

6. Cotyle modulaire de rotule selon une des revendications 1 à 5, **caractérisée par le fait que** les éléments de fixation (6) se rétrécissent en forme de coin (23) dans la direction d'insertion dans le corps de cotyle (7) et que les évidements (8) dans le corps de cotyle (7) sont adaptés au contour des éléments de fixation (6).

7. Cotyle modulaire de rotule selon une des revendications 1 à 6, **caractérisée par le fait que** le diamètre extérieur (12) de la cupule (2) est constant et que pour des diamètres intérieurs (11) différents du corps de cotyle (7) recevant la cupule (2), le diamètre extérieur (13) de la bague (5) est adapté au diamètre intérieur (11) du corps de cotyle (7), de telle sorte que les dimensions de l'interstice (14) soient toujours sensiblement identiques dans les différentes combinaisons possibles cupule (2)-corps de cotyle (7).

8. Cotyle modulaire de rotule selon une des revendications 1 à 7, **caractérisée par le fait qu'**il est prévu un outil (30) pour manipuler la cupule (2) montée dans une bague (5) pour l'insérer dans le corps de cotyle (7) et l'extraire de celui-ci.

9. Cotyle modulaire de rotule selon la revendication 8, **caractérisée par le fait que** l'outil (30) comporte deux éléments (31, 32) qui peuvent être déplacés axialement l'un par rapport à l'autre, que le premier élément, en tant que dispositif s'appui (31), prend appui sur le bord du corps de cotyle (7) et que le deuxième élément, en tant que dispositif de poussée et de traction (32) selon la manipulation prévue de la cupule (2), peut être positionné sur les éléments de fixation (6) de la cupule (2).

10. Cotyle modulaire de rotule selon la revendication 9, **caractérisée par le fait que** pour l'insertion de la cupule (2) dans le corps de cotyle (7), le dispositif de poussée et de traction (32) et le dispositif d'appui (31) peuvent être positionnés respectivement sur les éléments de fixation (6) et sur le bord du corps de cotyle (7), et que par un déplacement des deux éléments (31, 32) de l'outil (30) dans des directions opposées, une poussée peut être exercée sur la cupule (2) en direction (44) du corps de cotyle (7).

11. Cotyle modulaire de rotule selon la revendication 9, **caractérisée par le fait que** pour l'extraction d'une cupule (2) du corps de cotyle (7), le dispositif d'appui (31) peut être positionné sur le bord du corps de cotyle (7) et le dispositif de poussée et de traction (32) peut s'accrocher derrière les éléments de fixation (6) et que par coulissement des deux éléments (31, 32) de l'outil (30) en direction l'un de l'autre, la cupule (2) peut être extraite du corps de cotyle (7).
